(19) Europäisches Patentamt / European Patent Office / Office européen des brevets

(11) **EP 1 562 655 B1**

(12) **EUROPÄISCHE PATENTSCHRIFT**

(45) Veröffentlichungstag und Bekanntmachung des Hinweises auf die Patenterteilung:
**19.12.2012 Patentblatt 2012/51**

(21) Anmeldenummer: **03811335.3**

(22) Anmeldetag: **29.10.2003**

(51) Int Cl.:
***A61M 16/00*** (2006.01)

(86) Internationale Anmeldenummer:
**PCT/DE2003/003592**

(87) Internationale Veröffentlichungsnummer:
**WO 2004/045670 (03.06.2004 Gazette 2004/23)**

(54) **Verfahren zur Kompensation des Druckabfalls an einem Beatmungsschlauch, Beatmungsgerät sowie Speichermedium**

Method for compensating a pressure drop in a ventilator tube, ventilator and memory medium

Procédé de compensation d'une chute de pression dans un flexible respiratoire, appareil respiratoire et support d'enregistrement

(84) Benannte Vertragsstaaten:
**AT BE BG CH CY CZ DE DK EE ES FI FR GB GR HU IE IT LI LU MC NL PT RO SE SI SK TR**

(30) Priorität: **19.11.2002 DE 10253947**

(43) Veröffentlichungstag der Anmeldung:
**17.08.2005 Patentblatt 2005/33**

(73) Patentinhaber: **CareFusion Germany 234 GmbH 97204 Höchberg (DE)**

(72) Erfinder:
• **BAECKE, Martin**
  **06847 Dessau (DE)**
• **ANGER, Ewald**
  **97246 Eibelstadt (DE)**
• **REINSTAEDTLER, Juergen**
  **97082 Würzburg (DE)**

(74) Vertreter: **Hellmich, Wolfgang**
  **European Patent and Trademark Attorney**
  **Lortzingstrasse 9 / 2. Stock**
  **81241 München (DE)**

(56) Entgegenhaltungen:
| | |
|---|---|
| WO-A1-00/27457 | WO-A1-99/22793 |
| WO-A2-01/32069 | WO-A2-02/20076 |
| US-A- 5 551 419 | US-A- 5 694 923 |
| US-A- 6 085 747 | US-A- 6 105 575 |
| US-A1- 2002 005 197 | US-A1- 2002 023 644 |

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Beatmungsgerät, das geeignet ist, den Druckabfall an einem Beatmungsschlauch zu kompensieren und ein Speichermedium zur Speicherung eines entsprechenden Programms.

Bekannt sind Beatmungsgeräte oder Respiratoren zur maschinellen, künstlichen Beatmung bei allen Formen des Sauerstoffmangelzustands. Sie werden unter anderem für die Langzeitbeatmung eingesetzt. (Roche Lexikon Medizin, 4. Auflage, herausgegeben von der Hoffmann-La Roche AG und Urban & Fischer, Urban & Fischer, München, Stuttgart, Jena, Lübeck, Ulm, vgl. auch US 2002/005197 A1).

Bei frühen Beatmungsgeräten um 1950 wurden Scheibenwischermotoren als Antrieb für Blasebälge verwendet. Elektromotoren führten jedoch bei den damals verwendeten Ästhetika zu einer Explosionsgefahr. 1952 entwickelte Roger Manley vom Westminster-Krankenhaus in London ein Beatmungsgerät das ausschließlich von Gas angetrieben wurde. Dies war vier Jahrzehnte lang vor der Einführung elektronisch gesteuerte Beatmungsgeräte sehr populär. Seine Arbeitsweise war sehr einfach. Ein Gasfluss hob eine mit einem Gewicht beschwerte Blasebalgeinheit, die unter dem Einfluss der Schwerkraft immer wieder herunterfiel und dabei Beatmungsgase in die Lunge eines Patienten drückte. Der Beatmungsdruck konnte durch Verschieben des Gewichts auf den Blasebälgen eingestellt werden. Auch das Beatmungsvolumen konnte über eine Kurvenscheibe eingestellt werden, die die Auslenkung der Blasebälge begrenzte. Schließlich konnte der Restdruck nach Ende der Ausatmung eingestellt werden. Diese robuste Einheit beflügelte die Einführung von Überdruckbeatmungstechniken in Europa.

Volumengesteuerte Beatmung war in Europa erst 1971 mit dem SERVO 900 (Elema-Schönander) möglich.

Die WO 02/20076 A2 beschäftigt sich mit einem Steuerproblem bei der druckbasierten Beatmung über einen Doppelschlauch, d.h. die Expirationsluft wird im Beatmungsgerät gestaut um den Beatmungsdruck zu erzeugen. Hierzu wird ein einfaches Modell entworfen, das die Elastizität der Lunge des Patienten, die Elastizität der Beatmungsschläuche sowie den Fließwiderstand zwischen einem Y Stück der Beatmungsschläuche und dem distalen Ende eines Endotrachealschlauches berücksichtigt.

Eine besondere Form von Beatmungsgeräten, so genannten CPAP-Geräte werden eingesetzt, um obstruktive Atmungsstörungen während des Schlafens zu vermeiden. Ein solches Gerät ist aus der US 6,085,747 bekannt. Im Gegensatz zu älteren Beatmungsgeräten führt nur ein Schlauch zum Patienten. Ähnliche Einschlauch-Beatmungsgeräte werden inzwischen auch im Krankenhaus zur druckunterstützten Beatmung eingesetzt. Durch die Einsparung eines Schlauchs reduziert sich das Gewicht an der Atemmaske deutlich, was einen erhöhten Patientenkomfort und weniger Leckagen zur Folge hat. Aus historischen Gründen wird im Folgenden auf diese Art der Beatmung als CPAP referiert.

Obstruktive Atmungsstörungen führen zu Apnoen (Atemstillstand), durch die der Schlafende erwacht. Häufige Apnoen verhindern, dass der Schlafende in den erholsamen Tiefschlaf fällt. Menschen, die Apnoen während des Schlafens erleiden, sind deshalb tagsüber unausgeschlafen, was zu sozialen Problemen am Arbeitsplatz und im schlimmsten Fall zu tödlichen Unfällen, beispielsweise bei Berufskraftfahrern, führen kann.

Zur Behandlung von Apnoen wurde die CPAP (continuous positive airway pressure)-Therapie entwickelt, die in Chest. Volume No. 110, Seiten 1077-1088, Oktober 1996 und Sleep, Volume No. 19, Seiten 184-188, beschrieben wird. Ein CPAP-Gerät erzeugt mittels eines Gebläses einen positiven Überdruck bis zu etwa 30 mbar und appliziert diesen vorzugsweise über einen Luftbefeuchter, über einen Beatmungsschlauch und eine Gesichts- oder Nasenmaske in den Atemwegen des Patienten.

Dieser Überdruck soll gewährleisten, dass die oberen Atemwege während der gesamten Nacht vollständig geöffnet bleiben und somit keine Apnoen auftreten (DE 198 49.571 A1). Der erforderliche Überdruck wird auch als Therapiedruck $p_t$ bezeichnet. Er hängt unter anderem von dem Schlafstadium und der Körperposition des Schlafenden ab.

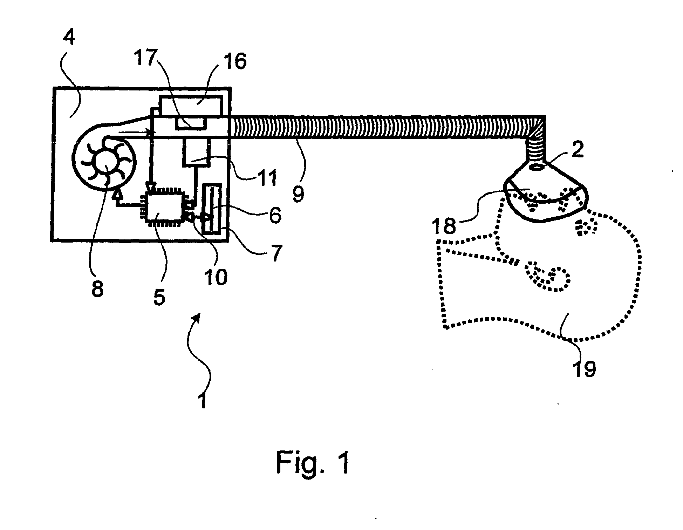

Fig. 1 zeigt ein CPAP-Gerät 1 im Therapieeinsatz. Das CPAP-Gerät 1 umfasst ein Gehäuse 4, einen Beatmungsschlauch 9 sowie eine Nasen- oder Gesichtsmaske 18. Das Gehäuse 4 enthält ein Gebläse 8, das auch als Verdichter, Lüfter, Ventilator oder Turbine bezeichnet wird. In der Nähe des Beatmungsschlauchanschlusses innerhalb des Gehäuses ist ein Drucksensor 11 zur Messung des von dem Gebläse erzeugten Überdrucks gegenüber dem Umgebungsdruck vorgesehen. Der gemessene Überdruck wird im Folgenden als Istdruck bezeichnet. Die von dem Gebläse 8 geförderte Luft wird über einen Beatmungsschlauch 9 der Gesichtsmaske 18 zugeführt, die der Patient 19 selbst trägt. In oder nahe der Gesichtsmaske 18 ist ein Ausatemöffnung 2 vorgesehen, durch die ein ständiger Luftstrom vom Beatmungsschlauch in die Umgebung stattfindet. Dieser Luftstrom sorgt dafür, dass die vom Patienten ausgeatmete Luft an die Umgebung abgeführt wird und verhindert, dass sich im Beatmungsschlauch 9 $CO_2$ anreichert. Ein Mikrocontroller 5 steuert die Drehzahl des Gebläses so, dass der vom Drucksensor 11 gemessene Istdruck mit einem Solldruck übereinstimmt. Ein ähnliches CPAP-Gerät ist aus der WO 01/32069 bekannt. Durch die Atmung des Patienten wird der Luftstrom durch den Schlauch und durch die Abblaseöffnung moduliert. Diese Modulation sorgt für eine Variation des Überdrucks in der Atemmaske des Patienten.

Bei allen bekannten CPAP-Geräten und in der Patentliteratur werden bisher zwei Verfahren verwendet, um den Druck in der Gesichtsmaske von CPAP-Geräten zu bestimmen.

Bei dem einen erfasst ein Drucksensor den Druck der zum Patienten geförderten Luft direkt im Gerät. Werksseitig wird für eine häufig verwendete Kombination von Beatmungsschlauch und Maske bei einem mittleren Volumenstrom die Druckdifferenz gemessen. Dieser Differenzwert wird vom im Gerät gemessenen Druck abgezogen und das Ergebnis als Maskendruck interpretiert. Zwangsläufig ergeben sich Fehler in der Druckeinstellung, weil der Luftstrom durch die Atmung ständig schwankt und damit der Druckverlust im Beatmungsschlauch variiert. Besonders lästig für die Patienten ist dabei, dass der Überdruck in der Gesichts - oder Nasenmaske beim Einatmen geringer ist als beim Ausatmen. Deshalb hat der Patient besonders bei dieser Form der Druckregelung das Gefühl, in der Summe gegen einen Widerstand atmen zu müssen.

Beim zweiten Verfahren wird der Druck beim Patienten über einen zusätzlichen Schlauch gemessen. Dieses Verfahren erzeugt einen stabilen Patientendruck, ist aber durch den zusätzlichen Schlauch nicht anwenderfreundlich und fehleranfällig. Beim anderen Verfahren, von denen eines in der WO 00/66207 beschrieben ist, erfolgt die Druckerfassung in der Nähe des Endes des Beatmungsschlauches vor der Maske oder in der Maske. Hierbei ist die Druckerfassung sehr genau, die Druckregelung gleicht auch Leckagen etc. aus. Die Druckmessung selbst kann mit einem Drucksensor erfolgen, dessen elektrische Anschlussdrähte mit dem Beatmungsschlauch zum Beatmungsgerät geführt werden müssen. Insbesondere bei Geräten von MAP wird ein separater dünner Schlauch von der Messstelle zu einem im CPAP-Gerät befindlichen Drucksensor geführt. Nachteilig bei diesen Varianten ist, dass spezielle Schläuche benötigt werden, um die Rückführung der Anschlussdrähte bzw. des Schlauches in das CPAP-Gerät zu ermöglichen. Diese Sonderanfertigungen sind teurer als normale Beatmungsschläuche. Ferner ist ihr Einsatz mit anderen CPAP-Geräten nur eingeschränkt möglich. Schließlich ist die Reinigung komplizierter.

Es ist Aufgabe der Erfindung, ein Beatmungsgerät sowie ein Speichermedium mit einem entsprechenden Programm anzugeben, bei dem der Druckabfall am Beatmungsschlauch kompensiert wird.

Diese Aufgabe wird durch die Lehre der unabhängigen Ansprüche gelöst.

Bevorzugte Ausführungsformen der Erfindung sind Gegenstand der Unteransprüche.

Vorteilhaft an der Erfindung ist, dass die Herstellkosten des CPAP-Geräts sinken, weil keine speziellen Beatmungsschläuche verwendet werden müssen.

Vorteilhaft am Messen des Luftflusses bei zwei unterschiedlichen Drücken ist, dass die beiden Parameter C und a in Gleichung 6 anhand von Messwerten bestimmt werden, so dass der während der Therapie für einen gemessenen Fluss bestimmte Druckabfall genauer dem tatsächlichen Druckabfall am Beatmungsschlauch entspricht.

Bei je mehr Drücken der Fluss während der Initialisierungsphase bei abgesetzter Beatmungsmaske gemessen wird, desto genauer ist während der Therapie die Druckkompensation.

Das Anpassen des Parameters C oder der Parameter C und a an eine Vielzahl von Druck- und Flusswerten reduziert den Einfluss möglicher Ausreißer bei den gemessenen Werten und vermeidet den Suchaufwand nach dem richtigen Intervall für eine abschnittsweise definierte Funktion.

Das Verwerfen von während der Initialisierung gemessenen Drücken reduziert den Speicheraufwand in vorteilhafter Weise. Durch das abschnittsweise Anpassen des Parameters C oder der Parameter C und a in Gleichung 6 an Intervalle, an deren unteren und oberen Ende sowohl Druck als auch Fluss gemessen wurden, führt zu einer sehr genauen Übereinstimmung zwischen dem berechneten und dem tatsächlichen Druckabfall am Beatmungsschlauch. Ausreißer bei der Druck- oder Flussmessung beeinflussen nur die angrenzenden Intervalle.

Das Kriterium bei einer nennenswerten Differenz zwischen Solldruck und Istdruck, die Initialisierungsphase abzubrechen, ist vorteilhaft, weil hierdurch sowohl der gesamte zur Verfügung stehende Flussbereich durch Messpunkte abgedeckt wird als auch vermieden wird, dass beim maximalen Fluss mehrere Messpunkte aufgenommen werden. Letzteres führt zu einer unnötig langen Initialisierung.

Das Überprüfen, ob der Parameter C in einem vernünftigen Bereich liegt schon während des Messens der Druck- und Flusswerte während der Initialisierungsphase und dem Abbrechen der Initialisierungsphase, wenn der Parameter C nicht in einem vernünftigen Bereich liegt, stellt beispielsweise sicher, dass der Patient die Maske nicht bereits während der Initialisierung aufgesetzt hat.

Die Verwendung eines gespeicherten Parameterwerts ist eine sinnvolle Rückfallposition, falls die Initialisierung nicht durchgeführt werden kann. Dies ist z.B. dann der Fall, wenn der Patient die Maske bereits bei ausgeschaltetem Gerät aufgesetzt hat.

Das Verwenden von Istdruckwerten anstatt von Solldruckwerten beim erfindungsgemäßen Beatmungsgerät vermeidet Fehler bei Abweichungen zwischen Ist - und Solldruck.

Im Folgenden wird eine bevorzugte Ausführungsform der Erfindung unter Bezugnahme auf die beiliegenden Zeichnungen näher erläutert. Dabei zeigen:

Fig. 1 ein CPAP-Gerät;

Fig. 2 ein Flussdiagramm eines erfindungsgemäßen Initialisierungsverfahrens; und

Fig. 3 ein Flussdiagramm eines erfindungsgemäßen Verfahrens zur Druckabfallkompensation an einem Beatmungsschlauch.

Wie oben erwähnt schwankt der Druckabfall am Beatmungsschlauch mit dem Luftfluss durch den Beatmungsschlauch. Der Luftfluss wiederum ändert sich während eines Atemzyklusses. Er ist insbesondere beim Einatmen hoch und beim Ausatmen niedrig und kann beim Ausatmen sogar sein Vorzeichen wechseln. Wird nun der vom Beatmungsgerät gelieferte Druck konstant gehalten, so ist der Druck in der Beatmungsmaske beim Ausatmen höher als beim Einatmen, was für den Patienten unangenehm ist. Hier schafft diese Erfindung dadurch Abhilfe, dass der gewünschte Therapiedruck $p_t$, der möglichst gleich dem Maskendruck $p_m$ sein soll um den Druckabfall am Schlauch korrigiert wird. Als Solldruck p wird die Summe aus Therapiedruck $p_t$ und Druckabfall $\Delta p$ eingestellt:

$$p = p_S + \Delta p \tag{1}$$

Der Druckabfall $\Delta p$ ist eine Funktion des Luftflusses:

$$\Delta p = f\left(\dot{V}\right) \tag{2}$$

Um den Luftfluss zu messen, ist das erfindungsgemäße CPAP-Gerät mit einem Luftflusssensor 16 ausgerüstet. Der Luftflusssensor 16 kann den Luftfluss anhand des Wärmeverlustes eines Heizdrahts 17 bestimmen. Wie oben erwähnt schwankt der Luftfluss mit der Atmung des Patienten. Der Druckabfall $\Delta p$ soll deshalb öfter während eines Atemzyklusses berechnet werden. Ein Atemzyklus dauert typischerweise zwischen 3 und 5 Sekunden, so dass der Druckabfall und der Solldruck mindestens zweimal pro Sekunde berechnet werden sollen.

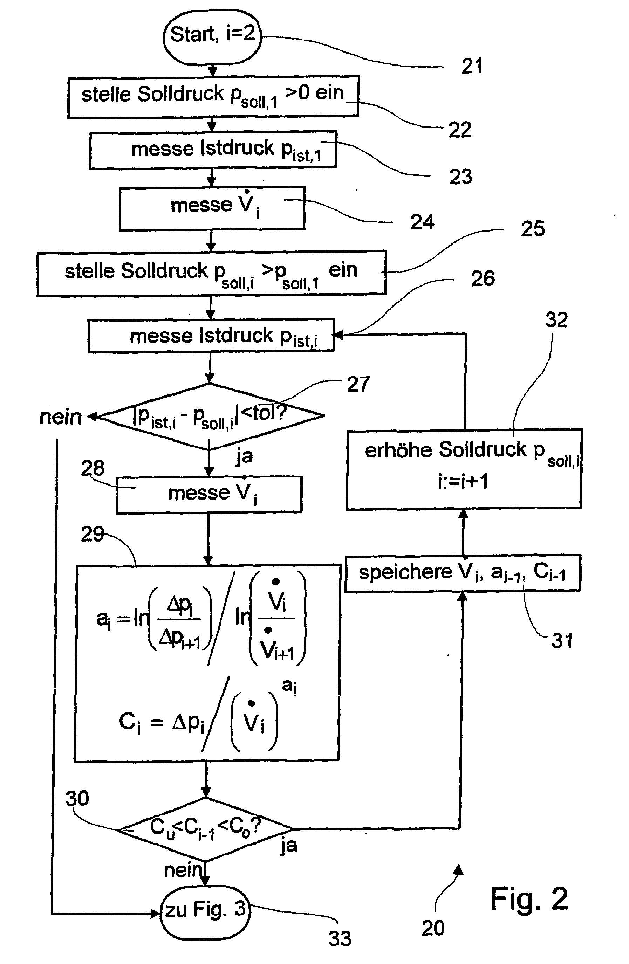

Gemäß der Erfindung wird nach Einschalten des Geräts und bevor der Patient 19 die Beatmungsmaske 18 aufsetzt, eine Initialisierung durchgeführt, deren Flussdiagramm in Fig. 2 dargestellt ist. Bei abgesetzter Maske ist der Maskendruck $p_m$ gleich dem Umgebungsdruck. Daraus ergibt sich, dass der am Drucksensor 11 gemessene Druck gleich dem Druckabfall $\Delta p$ am Beatmungsschlauch ist. Gemäß einer Ausführungsform wird während der Initialisierung der Solldruck in mehreren Stufen (Schritt 32) bis zu einem Maximalwert erhöht und jeder Stufe der Istdruck $\Delta pi$ und der Luftfluss $\dot{V}_i$ (Schritte 26, 28, 28) gemessen. Nach der Initialisierung können die gemessenen Druck- und Flusswerte $\Delta p_i$ bzw. $\dot{V}_i$ zur weiteren Verwendung gespeichert werden. Während der Therapie, wenn also der Patient die Beatmungsmaske 18 aufgesetzt hat, kann zu einem gemessenen Luftfluss $\dot{V}$ der am nächstliegende während der Initialisierung gemessene Luftfluss $\dot{V}_i$ gesucht werden, so dass sich der Druckabfall als $\Delta p_i$ ergibt. Es kann auch vorkommen, dass während der Ausatemphase negative Flüsse gemessen werden. In diesem Fall wird nach dem betragsmäßig möglichst gleich großen $\dot{V}_i$ gesucht und $\Delta p_i$ erhält das Vorzeichen des gemessenen Flusses. Nachteilig an diesem Verfahren ist, dass während der Initialisierung sehr viele Druck- und Flusswerte gemessen werden müssen, um den Druckabfall ausreichend genau bestimmen zu können.

Um mit weniger Druck- und Flusswerten auszukommen, kann zwischen den gemessenen Flusswerten beispielsweise durch lineare Interpolation gemäß Gleichung (3) der Druckabfall $\Delta p$ berechnet werden. Bevor jedoch Gleichung (3) angewendet wird, muss noch der Index i bestimmt werden, d.h. es muss das richtige Intervall für den gemessenen Fluss $\dot{V}$ gefunden werden.

$$\Delta p\left(\overset{\bullet}{V}\right) = \Delta p_i + \frac{\Delta p_{i+1} - \Delta p_i}{\overset{\bullet}{V}_{i+1} - \overset{\bullet}{V}_i}\left(\overset{\bullet}{V} - \overset{\bullet}{V}_i\right) \qquad (3)$$

wobei $\overset{\bullet}{V}_i \leq \overset{\bullet}{V} < \overset{\bullet}{V}_{i+1}$

Dabei nimmt der Faktor $\mathrm{sign}\left(\overset{\bullet}{V}\right)$ entweder den Wert 1 bei Luftstrom zum Patienten oder -1 bei Luftstrom vom Patienten an, um die Flussrichtung zu berücksichtigen.

Die lineare Interpolation ist lediglich ein mathematisches Verfahren, das zwar allgemein einsetzbar ist, jedoch die physikalischen Hintergründe des Luftflusses durch einen Schlauch ignoriert. Um die Anzahl von erforderlichen Messwerten $\Delta p_i$ und $\overset{\bullet}{V}_i$ weiter zu reduzieren, kann der Druckabfall an einem Schlauch wie dem Beatmungsschlauch 9 aus folgender Formel berechnet werden (Technische Strömungsmechanik 1, VEB Deutscher Verlag für Grundstoffindustrie, Leipzig):

$$\Delta p = \xi \frac{\rho}{2} v^a = \frac{\lambda l}{d} \bullet \frac{\rho}{2} v^a \bullet \mathrm{sign}(v) \qquad (4)$$

Dabei ist $\Delta p$ der am Schlauch abfallende Druck, $\xi$ ein Druckverlustbeiwert des Schlauches, $\lambda$ ein Rohrreibungswert des Schlauches, I die Länge des Schlauches, d der Durchmesser des Schlauches, p die Dichte des strömenden Mediums, also bei CPAP-Geräten ca. 1,2 kg/m$^3$ für Luft und v die über den Querschnitt gemittelte Strömungsgeschwindigkeit vom CPAP-Gerät in Richtung Maske. a hat den Wert 2 für turbulente Strömungen und 1 für laminare Strömungen. In der Praxis kann a auch Zwischenwerte annehmen, weil selten eine ideal-typische Strömungsform vorliegt. Bei den typischen Verhältnissen bei CPAP-Geräten liegen turbulente Strömungen vor, so dass a ≈ 2 ist. Die Gleichung (4) ist auch aus Strömungslehre, J. H. Spurk, 4. Auflage, Springerverlag, Berlin, 1996 bekannt, wobei hier $\lambda$ als Widerstandszahl bezeichnet wird. die gemittelte Strömungsgeschwindigkeit steht mit dem Luftstrom $\overset{\bullet}{V}$ in folgendem Zusammenhang:

$$\overset{\bullet}{V} = v \cdot \pi \cdot (d/2)^2 \qquad (5)$$

V selbst steht für ein Luftvolumen. Der Punkt bezeichnet die Ableitung nach der Zeit d/dt. $\overset{\bullet}{V}$ wird durch den Flusssensor 16 erfasst.

Setzt man (2) in (1) ein, erhält man folgende quadratische Abhängigkeit des Druckabfalls $\Delta p$ vom Luftstrom $\overset{\bullet}{V}$. Die Abhängigkeiten von $\lambda$, I, d sowie p wurden durch die Konstante C zusammengefasst, wobei C eine Kenngröße für den verwendeten Schlauch ist:

$$\Delta p = C \cdot \left|\overset{\bullet}{V}\right|^a \cdot \mathrm{sign}(\overset{\bullet}{V}) \qquad (6)$$

Gleichung (6) enthält die zwei Parameter C und a. Deshalb sind mindestens zwei Messpunkte bei unterschied-

lichen Drücken $\Delta p_i$ und $\Delta p_{i+1}$ sowie die entsprechenden Flusswerte $\dot{V}_i$ sowie $\dot{V}_{i+1}$ erforderlich, um die beiden Parameter zu bestimmen. In einer Ausführungsform können so die Parameter $a_i$ und $C_i$ für das Intervall i für einen Fluss $\dot{V}$ zwischen $\dot{V}_i$ und $\dot{V}_{i+1}$ gemäß Gleichungen (7) und (8) bestimmt werden. Der Druckabfall $\Delta p$ ergibt sich dann aus Gleichung (9).

$$a_i = \frac{\ln\left(\dfrac{\Delta p_i}{\Delta p_{i+1}}\right)}{\ln\left(\dfrac{\dot{V}_i}{\dot{V}_{i+1}}\right)} \tag{7}$$

$$C_i = \frac{\Delta p_i}{\left(\dot{V}_i\right)^{a_i}} \tag{8}$$

$$\Delta p = C_i \cdot \left|\dot{V}\right|^{a_i} \cdot \text{sign}(\dot{V}) \tag{9}$$

wobei $\dot{V}_i \leq \dot{V} < \dot{V}_{i+1}$

Wie man aus Gleichung (9) sieht, kommen hier die gemessenen Druckwerte des $\Delta p_i$ nicht mehr vor. Nach der Initialisierung müssen also lediglich die Flusswerte $\dot{V}_i$ sowie die Parameterwerte $C_i$ sowie $a_i$ zur weiteren Verwendung gespeichert werden (Schritt 31).

In einer anderen Ausführungsform kann der Parameter a auf einen Wert zwischen 1 und 2 gesetzt werden. Nur der Parameter $C_i$ wird für jedes Intervall getrennt aus Gleichung (8) berechnet. In dieser Ausführungsform wird nur der eine Wert a für alle $a_i$ sowie die Werte $C_i$ und $\dot{V}_i$ für die anschließende Therapiephase gespeichert.

Die während der Initialisierung gemessenen Druck- und Flusswerte $\Delta p_i$ bzw. $\dot{V}_i$ sollen möglichst den gesamten Flussbereich abdecken, ohne jedoch am oberen Ende mehrmals den gleichen Istdruck und Fluss zu messen, weil das Beatmungsgerät nicht genügend Luft liefern kann, so dass der Istdruck dem Solldruck nicht weiter folgen kann. Aus diesem Grund wird in Schritt 27 überprüft, ob sich vom Mikrocontroller 5 eingestellter Solldruck und vom Drucksensor 11 gemessener Istdruck um weniger als Toleranz tol unterscheiden. Ist dies der Fall, können noch weitere Druck- und Fluss-Paare in Schritten 28 und 26 gemessen werden. Ist dies nicht der Fall, wird die Initialisierung in Schritt 33 beendet.

Vorzugsweise werden bereits während der Initialisierung für jedes Messintervall in Schritt 29 die Parameter $a_i$ und $C_i$ oder nur $C_i$ nach Gleichungen (7) und (8) berechnet. So kann in Schritt 30 überprüft werden, ob der Parameter $C_i$ für ein Intervall i+1 in einem erlaubten Bereich zwischen einem unteren Wert $C_u$ und einem oberen Wert $C_o$ liegt. Wie oben erwähnt fasst der Parameter C die Widerstandszahl η, Schlauchlänge I sowie Schlauchdurchmesser d zusammen.

C stellt somit eine Art Widerstand des Schlauches dar. Setzt beispielsweise der Patient die Maske während der Initialisierung auf, steigt der berechnete Widerstandswert an, weil er sich jetzt als Serienschaltung des Schlauchs 9 mit der Ausatemöffnung 2 ergibt. Somit stellt Schritt 30 eine Plausibilitätskontrolle dar, ob noch richtige Werte gemessen werden. Wird die Initialisierung in Schritt 30

abgebrochen, so werden die bis dahin gemessenen Werte $\dot{V}_i$, $C_{1-1}$ und evtl. $a_{i-1}$ gespeichert und für höhere Flüsse die Werte einer vorausgehenden Initialisierung oder vorprogrammierte Standardwerte verwendet.

Da Gleichung (6) den Druckabfall an einem Schlauch in einem weiten Flussbereich in guter Näherung beschreibt, kann es ausreichen, dass lediglich bei zwei unterschiedlichen Druckwerten $\Delta p_i$ der Fluss $\dot{V}_i$ gemessen wird, um die Parameter a und C für den kompletten Flussbereich zu bestimmen. Keiner der Drücke oder Flüsse darf Null sein, da in Gleichung (7) nicht durch Null dividiert werden darf und der Logarithmus von Null nicht definiert ist. Auch müssen die Flüsse unterschiedlich sein, andernfalls ist ihr Verhältnis 1, der Logarithmus von 1 ist Null und durch Null darf nicht dividiert werden. Dies ist der Grund für die der Schleife vorgeschalteten Schritte 22 bis 24, in denen ein Druck-Flusswertepaar vor der Berechnung von $C_1$ gemessen wird.

In einer weiteren Ausführungsform kann der Parameter a werkseitig vorgegeben werden und lediglich der Fluss zu einem einzigen Istdruck $\Delta p$ bestimmt werden. Hieraus wird nach Gleichung (8) lediglich ein Parameter C berechnet.

In einer weiteren Ausführungsform können n Druck-Flusswertepaare $\Delta p_i$ und $\dot{V}_i$ gemessen werden, wobei $n \geq 3$ ist. An diese Wertepaare können die Parameter C und a oder nur C in Gleichung (6) angepasst werden. In dieser Ausführungsform werden Schwankungen in den Messwerten, die beispielsweise auf Rauschen zurückzuführen sind, ausgeglichen. Auch können die Messpunkte in mehrere Intervalle aufgeteilt werden, wobei jedes Intervall mindestens drei Messpunkte enthält, für jedes Intervall i kann entweder nur ein Parameter $C_i$ oder ein Parameter $C_i$ und ein Parameter $a_i$ angepasst werden.

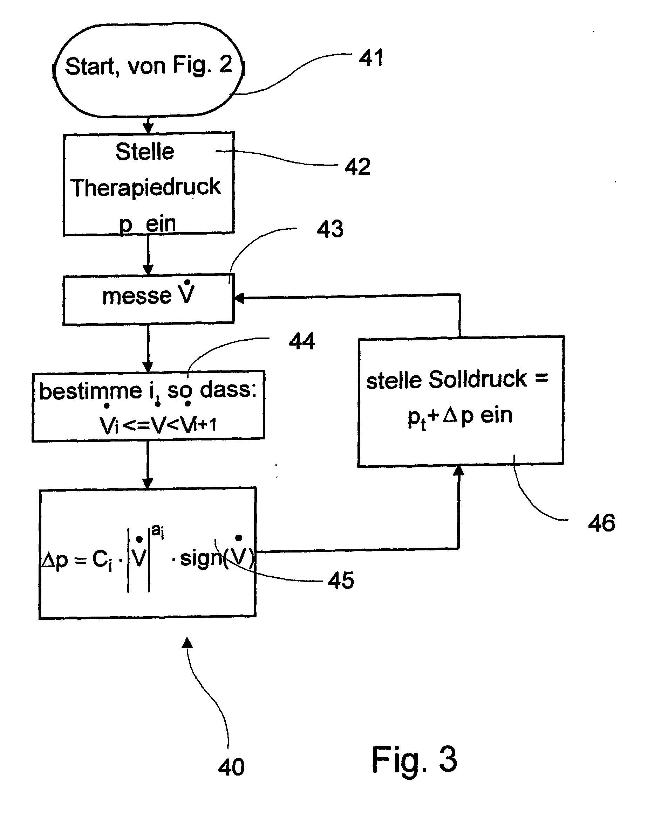

Fig. 3 zeigt das Verhalten eines Beatmungsgeräts, insbesondere eines CPAP-Geräts während der Therapie. Nach dem Start in Schritt 41 wird in Schritt 42 ein Therapiedruck $p_t$ eingestellt. Dieser kann, wie oben erwähnt, beispielsweise von einem Arzt vorgegeben werden. Bei diesem Therapiedruck wird in Schritt 43 durch Flusssensor 16 der Luftfluss gemessen. Ist die Funktion zur Berechnung des Druckabfalls $\Delta p$ aus dem Fluss $\dot{V}$ abschnittsweise wie in Gleichung (9) definiert, so wird in Schritt 44 das richtige Intervall i für den gemessenen Luftfluss $\dot{V}$ bestimmt. Überschreitet der Fluss $\dot{V}$ beispielsweise beim Einatmen den höchsten während der Initialisierung gemessenen Flusswert $V_n$, so werden die Parameter für das höchste Intervall n-1 verwendet. Anschließend wird in Schritt 45 mittels Gleichung (9) der Druckabfall bestimmt. Daraufhin wird ein neuer Solldruck aus Gleichung (1) in Schritt 46 berechnet. Anschließend wiederholt sich die Flussmessung in Schritt 43. Die aus den Schritten 43, 44, 45 und 46 bestehende Endlosschleife wird, wie oben erwähnt, pro Sekunde mindestens zweimal durchlaufen, damit der Solldruck entsprechend des Atemzyklus des Patienten schwankt, um den Druckanfall am Beatmungsschlauch zeitnah ausgleichen zu können. Neben der in Fig. 3 dargestellten Schleife ist, wie oben erwähnt, eine weitere Druckregelschleife vorhanden, die die Drehzahl der Turbine so regelt, dass der von Drucksensor 11 gemessene Druck dem eingestellten Solldruck p möglichst genau entspricht.

In dem erfindungsgemäßen CPAP-Gerät werden die in Fig. 2 und 3 dargestellten Schritte vom Mikrocontroller 5 ausgeführt. Um dessen Programmierung zu ändern, kann der Mikrocontroller über eine Datenleitung 10 mit einem Steckplatz 7 verbunden sein, in den ein Speichermedium 6 eingesteckt wird. Dies kann beispielsweise ein Memorystick oder eine PCMCIA-Karte sein. Der Steckplatz 7 kann jedoch ein IC-Sockel für einen PROM-Baustein (PROM: programable read only memory) sein. In einer weiteren Ausführungsform kann der Mikrocontroller 5 ein EPROM (electrically PROM) enthalten. In diesem Fall ist der Mikrocontroller selbst das Speichermedium, das ausgewechselt werden kann.

**Patentansprüche**

1. Beatmungsgerät mit:

   einem Lüfter (8),

einem Anschluss für einen Beatmungsschlauch (9), der mit dem Auslass des Lüfters (8) pneumatisch verbunden ist;

einem Flusssensor (16) zum Messen der vom Lüfter zum Anschluss für den Beatmungsschlauch (9) geförderten Luft; und

einer Steuerung (5), die mit dem Flusssensor (16) und dem Lüfter (8) elektrisch verbunden ist, wobei die Steuerung (5) bestimmt und geeignet ist, folgendes auszuführen:

Messen (24, 28) eines ersten Luftflusses mit dem Flusssensor (16) bei einem ersten Druck im Anschluss für den Beatmungsschlauch (9) und abgesetzter Beatmungsmaske;

Berechnen (29) eines Parameters in einer Funktion, die den Druckabfall am Beatmungsschlauch (9) in Abhängigkeit des Luftflusses liefert, wobei der Parameter für den ersten Luftfluss und ersten Druck berechnet wird;

Messen (43) des Luftflusses durch den Flusssensor (16) bei aufgesetzter Beatmungsmaske;

Berechnen eines Korrekturdrucks (44, 45) mittels der Funktion für den gemessenen Luftfluss; und

Einstellen eines Solldrucks (46), der als Summe aus gewähltem Maskendruck und dem Korrekturdruck ermittelt wurde.

2. Beatmungsgerät nach Anspruch 1, wobei die Steuerung (5) ferner bestimmt und geeignet ist,
einen zweiten Luftfluss (28) durch den Flusssensor (16) bei einem zweiten Druck (32) am Anschluss für einen Beatmungsschlauch (9) ungleich dem ersten Druck und abgesetzter Beatmungsmaske zu bestimmen und
den ersten Parameter und einen zweiten Parameter der Funktion zu berechnen, wobei die beiden Parameter aus dem ersten und zweiten Luftfluss und dem ersten und zweiten Druck berechnet werden.

3. Beatmungsgerät nach Anspruch 2, wobei die Steuerung (5) ferner bestimmt und geeignet ist,
eine Vielzahl von Drücken beim Anschluss des Beatmungsschlauchs (9) bei abgesetzter Beatmungsmaske am Beatmungsgerät einzustellen (32); und
den Luftfluss durch den Flusssensor (16) bei jedem der Vielzahl von Drücken zu messen.

4. Beatmungsgerät nach Anspruch 3, wobei die Steuerung (5) ferner bestimmt und geeignet ist, einen Parameter der Funktion so zu berechnen, dass die durch die Funktion zu den gemessenen Luftflüssen berechneten Drücke optimal mit den eingestellten Drücken übereinstimmen.

5. Beatmungsgerät nach einem der Ansprüche 1 bis 4, wobei die Steuerung (5) ferner bestimmt und geeignet ist, nach dem Berechnen des Parameters oder der Parameter nur den oder die Parameter zu speichern (31), nicht jedoch die bei abgesetzter Beatmungsmaske eingestellten Drücke.

6. Beatmungsgerät nach Anspruch 3, wobei die Steuerung (5) ferner bestimmt und geeignet ist, für jedes Intervall zwischen zwei bei abgesetzter Beatmungsmaske gemessenen Flüssen den Parameter für die Funktion aus den das Intervall begrenzenden Flüssen und den entsprechenden Drücken zu berechnen (29) und beim Berechnen des Korrektordrucks zunächst das Intervall zwischen zwei bei abgesetzter Beatmungsmaske gemessenen Drücken zu suchen (44) und in der Funktion den Parameter für das Intervall zu verwenden.

7. Beatmungsgerät nach einem der Ansprüche 3 bis 6, wobei die Steuerung (5) ferner bestimmt und geeignet ist, während des Einstellens der Vielzahl von Drücken die Höhe der Drücke monoton anzuheben und keine weiteren Drücke mehr einzustellen, wenn die Differenz zwischen einem vorgegebenen Solldruck und einem gemessenen Istdruck einen vorbestimmten Wert überschreitet.

8. Beatmungsgerät nach Anspruch 6 oder 7, soweit sich Anspruch 7 auf Anspruch 6 bezieht, wobei die Steuerung (5) ferner bestimmt und geeignet ist, die Parameter für ein Flussintervall zu berechnen (29), nachdem die Steuerung (5) die beiden Flüsse an den Intervallgrenzen in Zusammenwirken mit dem Flusssensor (16) bestimmt (28) und die entsprechenden Drücke am Beatmungsgerät eingestellt (32) hat und bevor die Steuerung (5) einen weiteren Druck einstellt (32), wobei die Steuerung (5) nur dann (30) einen weiteren Druck einstellt, wenn der Parameter in einem vorgegebenen Intervall liegt.

9. Beatmungsgerät nach einem der Ansprüche 1 bis 8, wobei die Steuerung (5) ferner bestimmt und geeignet ist, zu überprüfen, ob der berechnete Parameter in einem vorgegebenen Bereich liegt (30) und falls dies nicht der Fall ist, ein gespeicherter Parameterwert verwendet wird.

10. Beatmungsgerät nach einem der Ansprüche 1 bis 9, wobei das Beatmungsgerät ferner einen Drucksensor (11) umfasst, der mit der Steuerung (5) elektrisch verbunden ist, wobei die Steuerung (5) für das Berechnen des Parameters oder der Parameter durch den Drucksensor (11) gemessene Istdruckwerte (26) verwendet.

11. Speichermedium zur Verwendung mit einem Beatmungsgerät, das eine Mikzocontroller (5) zur Abarbeitung der in dem Speichermedium gespeicherten Befehle umfasst, wobei die Mikzocontroller (5) bei der Abarbeitung der im Speichermedium gespeicherten Befehle folgendes zur Kompensation des Druckabfalls an einem Beatmungsschlauch (9) durchführt:

Messen (24, 28) eines ersten Luftflusses bei einem ersten Druck im Anschluss für einen Beatmungsschlauch (9) und abgesetzter Beatmungsmaske;
Berechnen (29) eines Parameters in einer Funktion, die den Druckabfall am Beatmungsschlauch (9) in Abhängigkeit des Luftflusses liefert, wobei der Parameter für den ersten Luftfluss und ersten Druck berechnet wird;
Messen des Luftflusses (43) bei aufgesetzter Beatmungsmaske;
Berechnen eines Korrekturdrucks (44, 45) mittels der Funktion für den gemessenen Luftfluss;
Einstellen eines Solldrucks (46), der als Summe aus gewähltem Maskendruck und dem Korrekturdruck ermittelt wurde.

**Claims**

1. Ventilator, comprising:

   a fan (8),
   a connection for a ventilator tube (9), which is pneumatically connected to the outlet of the fan (8);
   a flow sensor (16) for measuring the air conveyed from the fan to the connection for the ventilator tube (9); and
   a controller (5), which is electrically connected to the flow sensor (16) and the fan (8), wherein the controller (5) is determined and adapted to carry out the following:

   measuring (24, 28) a first air flow by the flow sensor (16) at a first pressure in the connection for the ventilator tube (9) and with the respiratory mask taken off;
   calculating (29) a parameter in a function providing the pressure drop in the ventilator tube (9) in dependence on the air flow, wherein the parameter for the first air flow and the first pressure is calculated;
   measuring (43) the air flow by the flow sensor (16) with the respiratory mask being put on;
   calculating a correction pressure (44, 45) by means of the function for the measured air flow; and
   setting a target pressure (46), which was determined as sum of the selected mask pressure and the correction pressure.

2. Ventilator according to claim 1, wherein the controller (5) is further determined and adapted to determine a second air flow (28) by the flow sensor (16) at a second pressure (32) at the connection for a ventilator tube (9) unequal to the first pressure and with the respiratory mask taken off, and calculate the first parameter and a second parameter of the function, wherein both parameters are calculated from the first and second air flow and the first and second pressure.

3. Ventilator according to claim 2, wherein the controller (5) is further determined and adapted to set (32) a plurality of pressures at the ventilator on connecting the ventilator tube (9), with the respiratory mask taken off; and measure the air flow by the flow sensor (16) at each of the plurality of pressures.

4. Ventilator according to claim 3, wherein the controller (5) is further determined and adapted to calculate a parameter of the function such that the pressures calculated from the measured air flows by the function optimally correspond to the set pressures.

5. Ventilator according to one of claims 1 to 4, wherein the controller (5) is further determined and adapted to store (31), after the calculation of the parameter or the parameters, only the parameter(s), but not the pressures set with the respiratory mask taken off.

6. Ventilator according to claim 3, wherein the controller (5) is further determined and adapted to calculate (29) for each interval between two flows measured with the respiratory mask taken off the parameter for the function from the flows limiting the interval and the corresponding pressures and, when calculating the correction pressure, to search (44) at first the interval between two pressures measured with the respiratory mask taken off and use the parameter for the interval in the function.

7. Ventilator according to one of claims 3 to 6, wherein the controller (5) is further determined and adapted to monotonously increase the height of the pressures during the setting of the plurality of pressures and not to set further pressures if the difference between a predefined target pressure and a measured actual pressure exceeds a predetermined value.

8. Ventilator according to claim 6 or 7, in as far as claim 7 relates to claim 6, wherein the controller (5) is further determined and adapted to calculate (29) the parameters for a flow interval after the controller (5) has determined (28) the two flows at the interval boundaries in interaction with the flow sensor (16) and set (32) the corresponding pressures at the ventilator, and before the controller (5) sets (32) another pressure, wherein the controller (5) only sets (30) another pressure if the parameter is within a predefined interval.

9. Ventilator according to one of claims 1 to 8, wherein the controller (5) is further determined and adapted to check whether the calculated parameter is (30) within a predefined range and, if this is not the case, a stored parameter value is used.

10. Ventilator according to one of claims 1 to 9, wherein the ventilator further comprises a pressure sensor (11) which is electrically connected to the controller (5), wherein the controller (5) uses actual pressure values (26) measured by the pressure sensor (11) for calculating the parameter or the parameters.

11. Memory medium for use with a ventilator, comprising a microcontroller (5) for processing the commands stored in the memory medium, wherein the microcontroller (5) performs the following during the processing of the commands stored in the memory medium in order to compensate the pressure drop in a ventilator tube (9):

measuring (24, 28) a first air flow at a first pressure in the connection for the ventilator tube (9) and with the respiratory mask taken off;
calculating (29) a parameter in a function providing the pressure drop in the ventilator tube (9) in dependence on the air flow, wherein the parameter for the first air flow and the first pressure is calculated;
measuring the air flow (43) with the respiratory mask being put on;
calculating a correction pressure (44, 45) by means of the function for the measured air flow;
setting a target pressure (46), which was determined as sum of the selected mask pressure and the correction pressure.

**Revendications**

1. Appareil respiratoire pourvu de:

un ventilateur (8);
un raccord de flexible respiratoire (9) relié sur le plan pneumatique à la sortie du ventilateur (8);
un capteur de débit (16) servant à mesurer l'air extrait par le ventilateur jusqu'au raccord de flexible respiratoire (9); et
un élément de commande (5) relié sur le plan électrique au capteur de débit (16) et au ventilateur (8), l'élément de commande (5) étant défini et conçu pour mettre en oeuvre les points suivants:

mesure (24, 28) d'un premier débit d'air à l'aide du capteur de débit (16) à une première pression régnant dans le raccord de flexible respiratoire (9) et masque respiratoire retiré;
calcul (29) d'un paramètre dans une fonction établissant la chute de pression au niveau du flexible respiratoire (9) en fonction du débit d'air, le paramètre du premier débit d'air et la première pression étant calculés;
mesure (43) du débit d'air par le capteur de débit (16) lorsque le masque respiratoire est placé sur le visage;
calcul d'une pression de correction (44, 45) à l'aide de la fonction du débit d'air mesuré; et
réglage d'une pression théorique (46) calculée comme la somme de la pression choisie pour le masque et de la pression de correction.

**2.** Appareil respiratoire selon la revendication 1, l'élément de commande (5) étant en outre défini et conçu pour:

définir un deuxième débit d'air (28) à l'aide du capteur de débit (16) à une deuxième pression (32) régnant au niveau du raccord de flexible respiratoire (9) différente de la première pression et masque respiratoire retiré; calculer le premier paramètre et un deuxième paramètre de la fonction, les deux paramètres étant calculés à partir du premier et du deuxième débit d'air et de la première et de la deuxième pression.

**3.** Appareil respiratoire selon la revendication 2, l'élément de commande (5) étant en outre défini et conçu pour:

régler (32) au niveau de l'appareil respiratoire une pluralité de pressions au niveau du raccord du flexible respiratoire (9) lorsque le masque respiratoire est retiré; mesurer le débit d'air à l'aide du capteur de débit (16) à chacune des pressions parmi la pluralité de pressions.

**4.** Appareil respiratoire selon la revendication 3, l'élément de commande (5) étant en outre défini et conçu pour calculer un paramètre de la fonction, de façon à faire coïncider de façon optimale les pressions calculées par le biais de la fonction des débits d'air mesurés avec les pressions réglées.

**5.** Appareil respiratoire selon l'une quelconque des revendications 1 à 4, l'élément de commande (5) étant en outre défini et conçu pour n'enregistrer (31) après le calcul du paramètre ou des paramètres que le ou les paramètres et pour ne pas enregistrer les pressions réglées lorsque le masque respiratoire est retiré.

**6.** Appareil respiratoire selon la revendication 3, l'élément de commande (5) étant en outre défini et conçu pour calculer (29) le paramètre de la fonction à partir des débits délimitant l'intervalle et des pressions correspondantes pour chaque intervalle prévu entre deux débits mesurés lorsque le masque respiratoire est retiré et pour rechercher (44) d'abord lors du calcul de la pression de correction l'intervalle observé entre deux pressions mesurées lorsque le masque respiratoire est retiré puis pour utiliser le paramètre correspondant à l'intervalle dans la fonction.

**7.** Appareil respiratoire selon l'une quelconque des revendications 3 à 6, l'élément de commande (5) étant en outre défini et conçu pour relever de façon régulière le niveau des pressions pendant le réglage de la pluralité de pressions et pour arrêter de régler des pressions supplémentaires lorsque la différence entre une pression théorique prédéfinie et une pression réelle mesurée dépasse une valeur prédéfinie.

**8.** Appareil respiratoire selon la revendication 6 ou 7, avec la revendication 7 associée à la revendication 6, l'élément de commande (5) étant en outre défini et conçu pour calculer (29) les paramètres relatifs à un intervalle de débits après que l'élément de commande (5) a défini (28) les deux débits aux limites d'intervalle en interaction avec le capteur de débit (16) et réglé (32) les pressions correspondantes au niveau de l'appareil respiratoire et avant que l'élément de commande (5) n'applique une pression supplémentaire, l'élément de commande (5) ne réglant (30) une pression supplémentaire que lorsque le paramètre se situe dans un intervalle prédéfini.

**9.** Appareil respiratoire selon l'une quelconque des revendications 1 à 8, l'élément de commande (5) étant en outre défini et conçu pour vérifier (30) si le paramètre calculé se place dans une plage prédéfinie et si ce n'est pas le cas, pour utiliser une valeur de paramètre enregistrée.

**10.** Appareil respiratoire selon l'une quelconque des revendications 1 à 9, l'appareil respiratoire comprenant en outre un capteur de pression (11) relié sur le plan électrique à l'élément de commande (5), l'élément de commande (5) utilisant les valeurs de pression réelle (26) mesurées pour calculer le paramètre ou les paramètres à l'aide du capteur de pression (11).

**11.** Support d'enregistrement utilisé avec un appareil respiratoire, comprenant un microcontrôleur (5) servant à traiter les ordres enregistrés dans le support d'enregistrement, le microcontrôleur (5) exécutant les étapes suivantes lors du traitement des ordres enregistrés dans le support d'enregistrement en vue de compenser la chute de pression observée au niveau d'un flexible respiratoire (9):

mesure (24, 28) d'un premier débit d'air à une première pression régnant dans le raccord de flexible respiratoire (9) et lorsque le masque respiratoire est retiré; calcul (29) d'un paramètre dans une fonction établissant la chute de pression au niveau du flexible respiratoire (9) en fonction du débit d'air, le paramètre du premier débit d'air et la première pression étant calculés; mesure du débit d'air (43) lorsque le masque respiratoire est placé sur le visage;

calcul d'une pression de correction (44, 45) à l'aide de la fonction du débit d'air mesuré; et
réglage d'une pression théorique (46) calculée comme la somme de la pression choisie pour le masque et de la pression de correction.

Fig. 1

Start, i=2 — 21

stelle Solldruck $p_{soll,1} > 0$ ein — 22

messe Istdruck $p_{ist,1}$ — 23

messe $\dot{V}_i$ — 24

stelle Solldruck $p_{soll,i} > p_{soll,1}$ ein — 25

messe Istdruck $p_{ist,i}$ — 26

$|p_{ist,i} - p_{soll,i}| < tol?$ — 27

nein

ja

28 — messe $\dot{V}_i$

29

$$a_i = \ln\left(\frac{\Delta p_i}{\Delta p_{i+1}}\right) \Big/ \ln\left(\frac{\dot{V}_i}{\dot{V}_{i+1}}\right)$$

$$C_i = \Delta p_i \Big/ \left(\dot{V}_i\right)^{a_i}$$

30 — $C_u < C_{i-1} < C_o?$

ja

nein

erhöhe Solldruck $p_{soll,i}$

$i := i+1$ — 32

speichere $\dot{V}_i$, $a_{i-1}$, $C_{i-1}$ — 31

zu Fig. 3 — 33

20

Fig. 2

Start, von Fig. 2 — 41

Stelle Therapiedruck p ein — 42

messe $\dot{V}$ — 43

bestimme $i$, so dass: $\dot{V_i} \leq \dot{V} < \dot{V_i}+1$ — 44

$\Delta p = C_i \cdot \left| \dot{V} \right|^{a_i} \cdot \mathrm{sign}(\dot{V})$ — 45

stelle Solldruck = $p_t + \Delta p$ ein — 46

40

Fig. 3

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 2002005197 A1
- WO 0220076 A2
- US 6085747 A
- DE 19849571 A1
- WO 0132069 A
- WO 0066207 A

### In der Beschreibung aufgeführte Nicht-Patentliteratur

- Roche Lexikon Medizin. Urban & Fischer
- *Chest.,* Oktober 1996, 1077-1088
- *Sleep,* vol. 19, 184-188
- **J. H. SPURK.** Strömungslehre. Springerverlag, 1996